Europäisches Patentamt

European Patent Office

Office européen des brevets

(19)

(11) Publication number: **0 051 430**
**B1**

# (12) EUROPEAN PATENT SPECIFICATION

(45) Date of publication of patent specification: **24.07.85**

(21) Application number: **81305079.6**

(22) Date of filing: **27.10.81**

(51) Int. Cl.⁴: **H 05 G 1/60,** A 61 B 6/02, G 03 B 42/02

(54) X-Ray apparatus.

(30) Priority: **30.10.80 JP 152668/80**

(43) Date of publication of application:
**12.05.82 Bulletin 82/19**

(45) Publication of the grant of the patent:
**24.07.85 Bulletin 85/30**

(84) Designated Contracting States:
**DE FR GB IT NL**

(56) References cited:
**DE-A-2 062 430**
**DE-B-1 262 772**
**GB-A-2 034 967**
**US-A-3 244 878**
**US-A-3 424 901**
**US-A-3 684 354**

**ELECTROMEDICA, vol. 43, no. 1, 1975,
Erlangen, DE K. DUMMLING et al.:
"Stereofernsehen und Stereokinematographie
mit modernen Röntgenanlagen", pages 35-39**

(73) Proprietor: **Kabushiki Kaisha Toshiba
72, Horikawa-cho Saiwai-ku
Kawasaki-shi Kanagawa-ken 210 (JP)**

(72) Inventor: **Nishio, Kohsaku Patent Division
Tokyo Shibaura Denki K.K. 72 Horikawa-cho
Saiwai-ku Kawasaki-shi, Kanagawa-ken (JP)**

(74) Representative: **Batchellor, John Robert et al
BATCHELLOR, KIRK & EYLES 2 Pear Tree Court
Farringdon Road
London EC1R 0DS (GB)**

Courier Press, Leamington Spa, England.

# Description

This invention relates to X-ray apparatus and is aimed at providing such apparatus capable of performing stereoscopic, or mono-scopic X-ray cinematography, or X-ray fluoroscopy.

In the field of X-ray examination, and particularly in that utilising angiography, there is a great demand to enable an object, such as a patient, to be viewed three-dimensionally. Previously, single directional radiography has generally been performed in the diagnosis and treatment of, for example, congenital heart disease, valvular heart disease, and hepatic artery disease of the heart, where cardiovascular angiography is feasible; uni-directional radiography however shows nothing of a three-dimensional object other than a single plane, and it is extremely difficult to view a unidirectional image stereoscopically.

If two-directional radiography is performed in an attempt to improve the examination of the three-dimensional object, only two plane images which differ only in the radiographic direction are obtained and, again, a stereoscope is unobtainable.

In cardiovascular angiography, X-ray fluoroscopy is performed, while a contrasting medium is injected in the object to be observed and the resulting fluoroscopic images are photographed with a cinecamera. However since, in the conventional system, a different X-ray tube and a different cinecamera is used for each radiographic direction, the resulting images in each direction is a plane image. Consequently, it is difficult to obtain precise observation of cardiovascular systems and the like because of the impossibility of stereoscopic viewing. From DE—B—1 62 772 an X-ray apparatus for stereoscopic cinematography is known designed to radiate X-rays from two points independently, the apparatus comprising a converter for converting an X-ray image derived by means of X-rays to an optical image; an optical system for projecting the optical image including a half mirror, first and second cinecameras arranged to receive the optical image when the half mirror is in operative position, means for synchronising the drives of the cinecameras so that their shutters are opened alternatively and so that the shutters are never open simultaneously and a switching controller which is designed to control that X-rays are radiated from the points alternatively in synchronisation with the alternatively open shutters of the cinecameras. From DE—A—2 062 430 an X-ray apparatus for stereoscopic cinematography is known comprising means alternatively emitting X-rays from two points, a converter, an optical system, a cinecamera and a television camera, also arranged to receive the optical image projected by the optical system. From GB—A—2034967 a stereoscopic X-ray device is known employing an X-ray tube designed to radiate X-rays from at least two focal points independently.

In the present invention, an X-ray tube designed to radiate X-rays independently from at least two focal points is employed with an optical system for independently projecting images derived from those focal points, so that stereographs may be obtained.

Thus, the present invention provides X-ray apparatus employing an X-ray tube designed to radiate X-rays from at least two focal points independently, the apparatus comprising a converter for converting an X-ray image derived by means of X-rays from the tube to an optical image; an optical system for projecting the optical image, the system including a first half-mirror and a second half-mirror which is arranged to receive light from the location of the first half-mirror, each of the half-mirrors being movable between an operative position in the light path and an inoperative position out of the light path; first and second cinecameras arranged to receive the optical image when the first half-mirror and second half-mirror respectively is in operative position the half-mirrors having reflectivities such that about the same quantum of light is transmitted to both cinecameras; means for synchronising the drives of the cinecameras so that their shutters are opened alternatively and so that the shutters are never open simultaneously; a television camera also arranged to receive the optical image projected by the optical system; mode setting equipment to select a stereoscopic cinematography mode, a mono-scopic cinematography mode, or a fluoroscopy mode; radiographic control means controlled by the setting equipment for controlling the drive of the cinecameras, and for generating X-ray exposure switching signals synchronised with the shutter movements of the cinecameras; and a switching controller for the X-ray tube which controller is operated by the switching signals and which is designed to control the X-ray tube so that X-rays are radiated from the focal points alternately in synchronism with the alternately open shutters of the cinecameras when the stereoscopic cinematography mode is selected.

By a "half-mirror" is meant a mirror which is part-transmissive and part-reflective.

The invention will be more readily understood by way of example from the following description of X-ray apparatus in accordance therewith, reference being made to the accompanying drawings, in which

Figure 1 is a block diagram, schematically illustrating the apparatus,

Figure 2 shows in axial section the optical system of the X-ray apparatus of Figure 1,

Figure 3 is a circuit diagram illustrating one form of the X-ray switching controller of Figure 1, and

Figures 4(a) to (f) are timing charts explaining the operation of the apparatus.

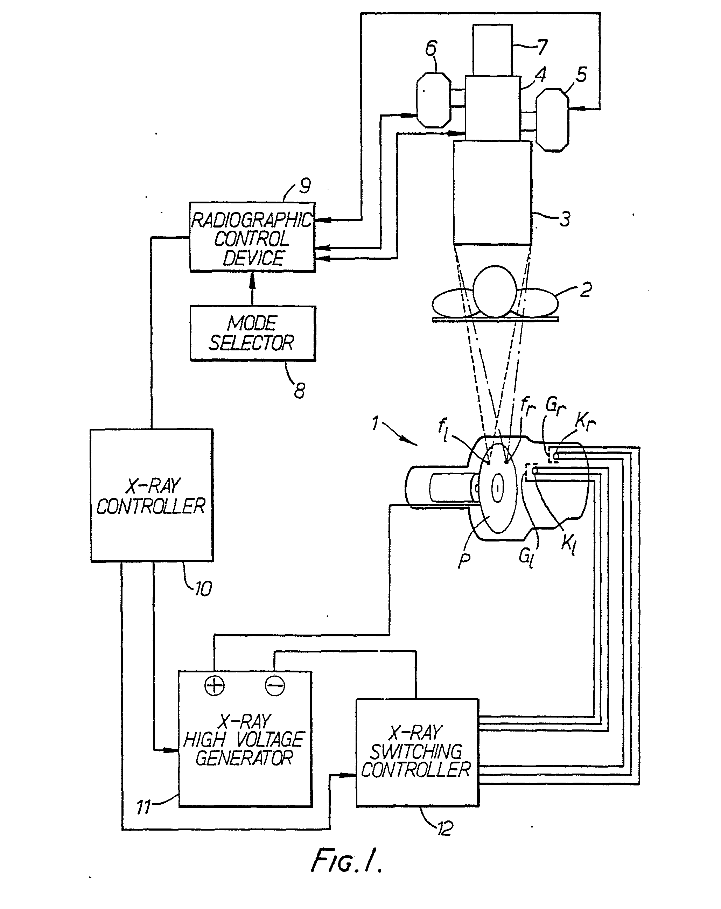

Referring to Figure 1, an X-ray tube 1 for stereoscopic radiography has a conical trapezoid shaped rotating anode P, and two cathodes Kr and Kl spaced apart by a distance corresponding to the average human inter-ocular distance, and facing the oblique part of anode P. Hereinafter, the suffixes r and l are in conjunction with the reference numerals of the drawings relate to the right and left eyes, respectively. Grids Gr and Gl are interposed between the cathodes Kr and Kl and the rotating anode P and are employed for X-ray exposure control. The potentials on the two grids are alternately altered to cause X-ray radiation alternatively from the corresponding anode parts during the times when electrons are emitted from the respective cathodes Kl and Kr. The two beams of electrons from the cathodes impinge upon the portions of the oblique anode P facing the cathodes, to define two focal points fl and fr from which a pair of X-ray beams are radiated. In this way, two X-ray beams are generated, spaced laterally at the inter-ocular distance. The X-ray tube 1 as described above is disclosed in U.S. Patent No. 4287420, for example.

An object 2 such as a patient is interposed between the X-ray tube 1 and an image intensifier 3 which receives on its input face the X-ray beams after passing through the object 2, and forms light images. Optical system 4 is connected to the output of intensifier 3 and projects the optical output image as will be described. Cinecameras 5 and 6 are attached to the sides of the optical system 4 to photograph optical images directed thereto to the optical system. The cinecameras 5 and 6 are synchronously driven, so that their shutters open alternately, and so that the shutters are never open together. Generally each shutter is formed by a disc having a window at a given angular position; by rotating the shutter one image per frame is photographed on the cinefilm, the movement of which is synchronised with the shutter movement. In that case, when stereoscopic radiography is to be performed, the shutters of the cinecameras are arranged 180° out of phase. Cinecameras 5 and 6 issue a series of shutter motion detection signals when their shutters are open, the outputs being employed for exposure control of the X-ray tube 1. A television camera 7 converts the optical image from the intensifier 3 to an electrical image signal and it delivers the latter signal to a television monitor (not shown).

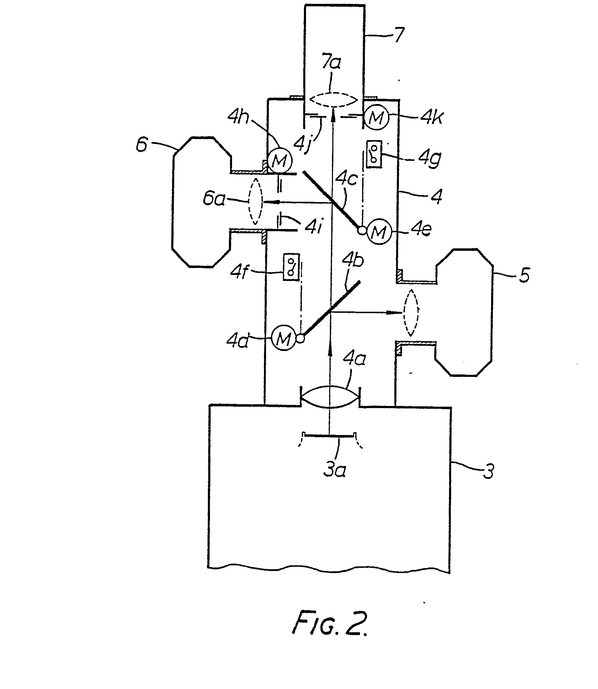

The optical system 4 and its peripherals are shown in detail in Figure 2. The optical system consists of a lightproof enclosure attached to the image intensifier 3 and having a lens 4a adjacent to the outward face 3a of the intensifier 3. Television camera 7 with its lens 7a is attached to the other end of the enclosure, coaxially with lens 4a. Cinecameras 5 and 6 are attached to opposite sides of the optical system 4 and at different distances from lens 4a; their optical axes are at right angles to the axis of lens 4a.

Half-mirrors 4b and 4c are mounted within the enclosure and opposite the respective cinecameras 5 and 6. Each half-mirror 4b or 4c can be moved by a motor 4d or 4e respectively between an operative position shown in full line, in which the half-mirror lies at an angle of 45° to the axis of lens 4a, and an inoperative position shown in chain line in which the half-mirror lies parallel to, and spaced from, the optical axis. When in its operative position, each half-mirror is effective to deflect a part of the incident light to the associated cinecamera.

Half-mirror 4b pertaining to cinecamera 5 is 50% reflective and 50% transmissive, while half-mirror 4c pertaining to cinecamera 6 is 90% reflective and 10% transmissive. Position detectors 4f and 4g are located in the neighbourhood of the half-mirrors 4b and 4c and detect whether the respective half-mirror is in its operative position or in its inoperative position.

An optical diaphragm 4i is driven by a motor 4h to control the amount of incident light to lens 6a of cinecamera 6, and compensates for movement of half-mirror 4b: when half-mirror is moved into its inoperative position, diaphragm 4i is operated to decrease the incident light, and vice versa. Diaphragm 4i may be constituted by the cinecamera's own diaphragm, provided that the latter can be externally controlled.

A second optical diaphragm 4j is provided to control the quantum of light to television camera 7 and is driven by motor 4k in accordance with the positions of half-mirrors 4b and 4c, in the manner of diaphragm 4i.

Reverting to Figure 1, a mode selector 8 determines the mode in which the apparatus is to operate, i.e. whether in the stereoscopic cinematography mode, the mono-scopic cinematography mode or the fluoroscopy mode. A radiographic control device 9 which receives signals from selector 8, the cinecamera shutter drives and the detectors 4f and 4g controls the motors 4d, 4e, 4h and 4k, the drives of cinecameras 5 and 6 when stereoscopy mode is selected, the drive of cinecamera 6 only when the mono-scopic mode is selected, and the X-ray tube 1.

An X-ray controller 10 controlled by control device 9 emits signals to X-ray high voltage generator 11 to set the X-ray tube voltage and current according to whether the fluoroscopy mode or the radiographic mode is selected. In addition, controller 10 emits signals to X-ray switching controller 12 which in turn controls the emission of the beams from the focal points fr and fl. Thus, the "right" beam from focal point fr is generated when the shutter of cinecamera 5 is open and the "left" X-ray beam from focal point fl is generated when the shutter of cinecamera 6 is open.

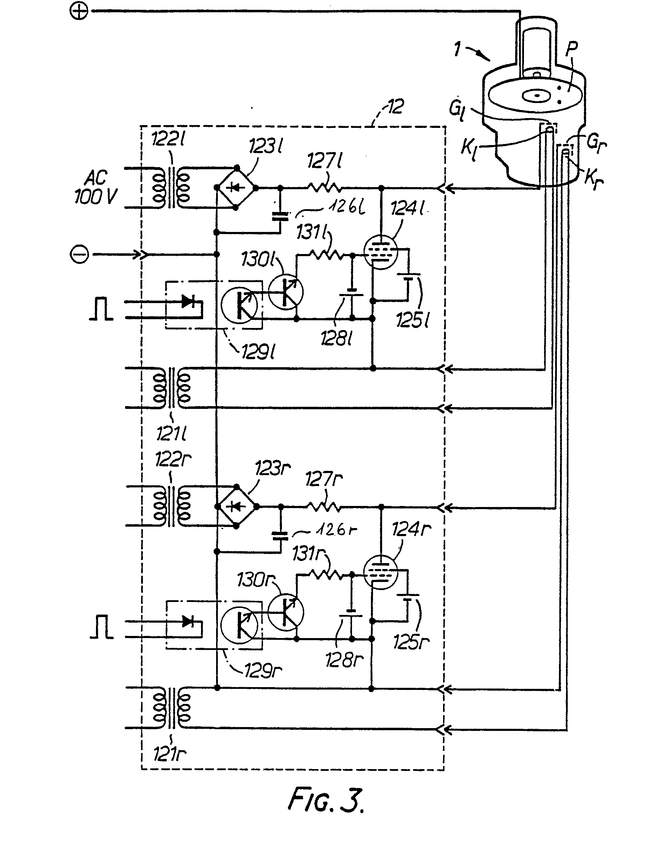

The X-ray tube switching controller 12 is shown in greater detail in Figure 3, in which the signs + and − designate input terminals which receive respectively the positive and negative high voltage outputs from the generator 11. Controller 12 includes two identical control circuits, one for the "right" X-ray beam and the other for the "left" X-ray beam. As the two circuits are identical, only one will be described, although both are shown in Figure 3 with the suffixes r and l.

A filament heating transformer 121 receives an output from generator 11 and generates a heating voltage corresponding to its input, that voltage being applied to the corresponding cathode K of the X-ray tube 1. Transformer 122 for producing a grid potential is connected to a power source and generates a variable voltage at its output. Rectifier circuit 123 performs full rectification to obtain a d.c. voltage of negative polarity. Tetrode 124 for the exposure switching control of the X-ray tube is connected between the corresponding grid and cathode of the X-ray tube 1. A power supply source 125 is connected between the cathode and second grid of tetrode 124 and applies to the second grid a positive bias to bring the internal resistance of the tetrode to an optimum value. Condenser 126 is connected between the output terminals of the rectifier circuit 123 and a resistor 127 is connected between the rectifying circuit 123 and the corresponding grid of tube 1. Potential source 128 is connected between the cathode and first grid of tetrode 124 to bias negatively the first grid to an optimum value.

A photo-coupler 129 is rendered active on receipt of the X-ray exposure switching control output from controller 10 and its output activates a switching transistor 130 having its emitter connected to the negative terminal of potential source 128 through resistor 131 and its collector to the positive terminal of that source.

The high voltage output from generator 11 is applied between the anode and the cathodes of tube 1, being applied to the cathodes through the filament heating transformers 121. The negative potential of rectifying circuit 123 is applied to the respective grid of tube 1 through resistor 127, and the positive potential to the respective cathode, so as to apply negative bias to the grid G and to hold the tube 1 in the cut-off state. Although the output voltage of rectifying circuit 123 is supplied to tetrode 124 as the tube voltage, the tetrode is inversely biased between its primary grid and cathode and is normally in its cut-off state.

Subsequently, when photo-coupler 129 receives a control output from controller 10, transistor 130 is rendered conducting, to close the loop formed by bias source 128, transistor 130, and resistor 131. The resulting voltage drop across resistor 131 increases the potential on the first grid of tetrode 124 and the tetrode is rendered conducting. Current now flows through resistor 127, the potential on the respective grid G of tube 1 is increased, electrons flow from the respective cathode K to the anode P, and an electron beam is emitted from the respective focal point fl or fr.

When the input to the photo-coupler 129 disappears, transistor 130 is rendered non-conducting causing tetrode 124 to be cut-off. Negative bias is again applied to the respective grid K of tube 1 and the X-ray beam from the corresponding focal point of the anode ceases. In this way, it is possible to control the X-ray exposures corresponding to the right and left eyes by controlling the photo-couplers 129r and 129l by the X-ray controllers 10.

The operation of the optical system will now be described:

Mode selector 8 is initially operated to select one of the three modes.

Stereoscopic cinematography
Selector 8 applies an appropriate instruction to control device 9 which, in turn, controls motors 4d and 4e as required to move the half-mirrors 4b and 4c into the optical path, as shown in full line in Figure 2; the control of the two motors is determined by the outputs of the detectors 4f and 4g, so that each motor is operated only if the respective half-mirror is in the inoperative position. A control output is also applied from control device 9 to each of the motors 4h and 4k to open the diaphragms 4i and 4j to their settings corresponding to the half-mirrors being in their operative positions. In this way, the optical system 4 is set for stereoscopic cinematography.

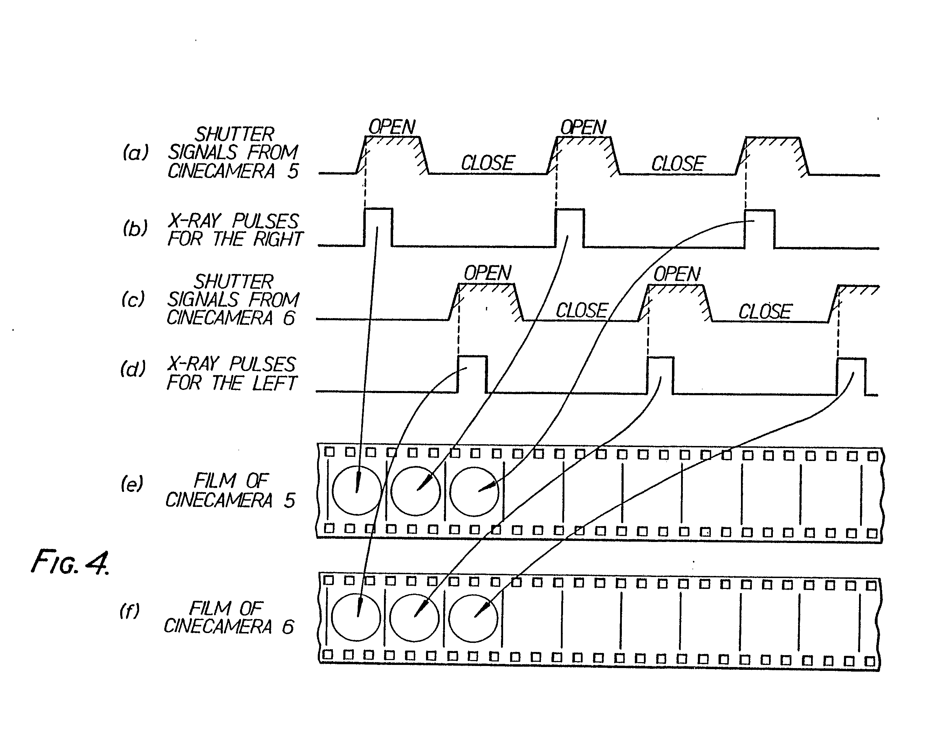

X-ray controller 10, under the control of a further signal from control device 9, sets the tube voltage and current for radiography. At the same time, a drive signal is delivered to cenecameras 5 and 6 to initiate shutter movement and film movement. As before mentioned, the shutters of the two cinecameras are displaced by 180°, so that when the shutter of one is open, that of the other is closed. Indication signals synchronised with the shutter opening times of cinecameras 5 and 6 are issued to control device 9, as shown in Figures 4(a) and 4(c) respectively. Control device 9 generates X-ray exposure switching output signals in synchronism with the signals from the cinecameras. Assuming that cinecamera 5 is to receive images corresponding to the right eye and cinecamera 6 images corresponding to the left eye, on receipt of an indication signal from cinecamera 5, control device 9 transmits an X-ray exposure switching control output to controller 10, which sends a signal to the photo-coupler 129r in the X-ray tube switching controller 12. That results in an X-ray beam being emitted from focal point fr of the X-ray tube 1, as described above; the pulses of X-ray emissions from point fr are shown in Figure 4(b). The resulting X-ray image of the object is received on the input face of intensifier 3 and is converted to an optical image which is directed to the optical system 4. 50% of the light is diverted by half-mirror 4b to cinecamera 5, which records the right-eye pictures of the object 2 as shown in Figure 4(e).

The 50% of the incident light transmitted by half-mirror 4b is received by the second half-mirror 4c which diverts 90% towards cinecamera 6, i.e. 45% of the initial quantum of light transmitted from the intensifier 3. However, as cinecamera 6 has its shutter closed, no exposure is made. The light transmitted through mirror 4c (5% of the initial quantum) is received by television camera 7 which displays the image on the monitor.

Similarly, when the shutter of cinecamera 5 closes and the shutter of cinecamera 6 opens, the resulting indication signals from the cinecameras results in photo-coupler 129r being cut off and photo-coupler 129l being rendered inactive, so that the "right" X-ray beam from focal point fr is cut off and the "left" X-ray beam from focal point fl is initiated. The resulting light beam is diverted by half mirrors 4b and 4c as before but, in this case, the shutter of cinecamera 5 is closed so that no exposure is made, whereas the shutter of cinecamera 6 is open and photographs the "left" pictures of the object. Television camera 7 again receives and displays the light transmitted by half-mirror 4c.

Until a stop instruction is issued, the "right" and "left" operations as described above are alternately repeated. In the result, cinecameras 5 and 6 produce "right" and "left" pictures of the object, which can be viewed subsequently in a stereoscope to obtain a three-dimensional view of the object.

## Mono-scopic cinematography

Under the control of selector 8, control device causes half-mirror 4b to be moved into the inoperative position shown in chain in Figure 2. Signals are applied to the motors 4h and 4k to adjust diaphragms 4i and 4j to alter the opening settings to values corresponding to the increased quantum of light reaching half-mirror 4c, due to half-mirror 4b being rendered inoperative. Subsequently, only cinecamera 6 is actuated and only the "left" grid Gl positively biased in order to cause pulses of X-ray to be emitted from focal point fl. The operation is otherwise as described above in relation to stereoscopic radiography, it being understood that half-mirror 4c transmits 90% of the initial light to cinecamera 6 and 10% to television camera 7. The amounts of light received through the shutter of cinecamera 6 is however compensated by diaphragm 4i, while diaphragm 4j of the television camera has a similar function.

## Fluoroscopic mode

Control device 9 delivers signals to motors 4d and 4e to bring both half-mirrors to their inoperative positions, if they are not already so positioned. Similarly, optical diaphragm 4j of television camera 7 is adjusted to compensate for the increased amount of light passing towards the television camera. Further, X-ray controller 10 is controlled to set the voltage and current of tube 1 to the values for fluoroscopy. Next, a signal for positively biasing the "right" grid Gr is applied to X-ray tube switching controller 12 from controller 10 to cause the X-ray beam to be emitted from focal point fr. The resulting X-ray image is converted by intensifier 3 to an optical image, which is led to television camera 7 to display it on the monitor.

## Claims

1. X-ray apparatus employing an X-ray tube (1) designed to radiate X-rays from at least two focal points (fl, fr) independently, the apparatus comprising a converter (3) for converting an X-ray image derived by means of X-rays from the tube to an optical image; an optical system (4) for projecting the optical image, the system including a first half-mirror (4b) and a second half-mirror (4c) which is arranged to receive light from the location of the first half-mirror, each of the half-mirrors being movable between an operative position in the light path and an inoperative position out of the light path, first and second cinecameras (5, 6) arranged to recive the optical image when the first half-mirror and second half-mirror respectively is in operative position, the half-mirrors having reflectivities such that about the same quantum of light is transmitted to both cinecameras; means for synchronising the drives of the cinecameras so that their shutters are opened alternately and so that the shutters are never open simultaneously; a television camera (7) also arranged to receive the optical image projected by the optical system; mode setting equipment (8) to select a stereoscopic cinematography mode, a mono-scopic cinematography mode, or a fluoroscopy mode; radiographic control means (9) controlled by the setting equipment for controlling the drive of the cinecameras, and for generating X-ray exposure switching signals synchronised with the shutter movements of the cinecameras; and a switching controller (12) for the X-ray tube which controller is operated by the switching signals and which is designed to control the X-ray tube so that X-rays are radiated from the focal points alternatively in synchronism with the alternately open shutters of the cinecameras when the stereoscopic cinematography mode is selected.

2. X-ray apparatus as claimed in claim 1, in which each of the second cinecamera and the television camera has, or is associated with, an adjustable diaphragm (4i, 4j) controlling the level of light entering the respective camera, and the radiographic control means is arranged to control the diaphragms according to the positioning of the half-mirrors.

3. X-ray apparatus as claimed in claim 1 or claim 2, in which the switching controllers includes an independent X-ray tube grid control circuit for independently controlling the potential on each of the two grids of the X-ray tube in response to the switching signals, in order to perform on-off control of the radiations from the two focal points.

4. X-ray apparatus as claimed in claim 3, in which each X-ray grid control circuit includes a tetrode ($124_l$, $124_r$).

5. x-ray apparatus as claimed in any one of the preceding claims, in which the converter is an image intensifier.

## Revendications

1. Appareil à rayons X utilisant un tube à rayons X (1) conçu pur émettre des rayons X, de façon indépendante, par, au moins, deux points focaux (fl, fr), installation qui comprend un convertisseur (3) pour convertir une image radiologique produite au moyen des rayons X provenant du tube en une image optique; un système optique (4) pour projeter l'image optique, les système incluant un premier miroir semi-réfléchissant (4b) et un second miroir semi-réfléchissant (4c) qui est arrangé pour recevoir la lumière de l'implacement du premier miroir, chacun des miroirs semi-argentés pouvant être déplacé entre une position active dans laquelle il est placé sur le trajet de la lumière, et une position inactive située en dehors de ce trajet; une première et une seconde caméras cinématographiques (5, 6) arrangées pour recevoir l'image optique quand respectivement le premier miroir semi-argenté ou le second miroir semi-argenté est dans sa position active, les miroirs semi-argentés ayant des réflectivités telles qu'environ la même quantité de lumière est transmise aux deux caméras cinématographiques; des moyens pour synchroniser les entraînements des caméras de façon que leurs obturateurs s'ouvrent alternativement et de manière que ces obturateurs ne soient jamais ouverts en même temps; une caméra de télévision (7) arrangée, elle aussi, pour recevoir l'image optique projetée par le système optique; un sélecteur de mode (8) permettant de choisir entre un mode cinématographique stéréoscopique, un mode cinématographique monoscopique ou un mode fluoroscopique; des moyens de commande radiographiques (9) commandés par les moyens de sélection pour régler l'entraînement des caméras cinématographiques et pour engendrer des signaux de commutation d'exposition synchronisés avec les mouvements des obturateurs des caméras; et, une commande de commutation (12) pour le tube à rayons X et qui est elle-même commandée par les signaux de commutation et est conçue pour commander le tube à rayons X de façon que les rayons X sont émis alternativement par les points focaux de celui-ci en synchronisme avec l'ouverture alternative des obturateurs des caméras cinématographiques, quand le mode cinématographique stéréoscopique a été sélectionné.

2. Appareil à rayons X selon la revendication 1, caractérisé en ce que la seconde caméra cinématographique et la caméra de télévision possèdent ou sont associées à un diaphragme réglable (4i, 4j) qui règle l'intensité de la lumière pénétrant dans la caméra correspondante, les moyens de commande radiographiques étant conçus pour régler les diaphragmes conformément à la position des miroires semi-argentés.

3. Appareil à rayons X selon la revendication 1 ou 2, caractérisé en ce que les commandes de commutation comprennent un circuit de commande indépendant de la grille du tube à rayons X, afin de régler séparément le potentiel sur chacune des deux grilles du tube à rayons X en fonction des signaux de commutation, afin de procéder à une commande de marche et d'arrêt des radiations issues des deux points focaux.

4. Appareil à rayons X selon la revendication 3, caractérisé en ce que chaque circuit de commande de grille du tube à rayons X contient une tétrode (124$_l$, 124$_r$).

5. Appareil de rayons X selon l'une quelconque des revendications précédentes, dans laquelle le convertisseur est un amplificateur de luminance ou de brillance.

**Patentansprüche**

1. Röntgengerät mit einer Röntgenröhre (1) zur Ausstrahlung von Röntgenstrahlen aus wenigstens zwei unabhängigen Brennpunkten ($f_l$, $f_r$), gekennziechnet durch einen Konverter (3) zur Umwandlung eines Röntgenstrahlbildes, das mit Hilfe von Röntgenstrahlen der Röntgenröhre erzeugt wird, in ein optisches Bild, durch ein optisches System (4) zur Abbildung des optischen Bildes, wobei das System einen ersten halbdurchlässigen Spiegel (4b) und einen zweiten halbdurchlässigen Spiegel (4c) aufweist, welcher so angeordnet ist, daß er Licht vom ersten halbdurchlässigen Spiegel empfängt, und wobei die halbdurchlässigen Spiegel zwischen einer Betriebsposition im Lichtweg und einer Außerbetriebsstellung außerhalb des Lichtweges bewegbar sind, durch erste und zweite Kinokameras (5, 6) die das optische Bild empfangen, wenn der erste halbdurchlässige Spiegel bzw. der zweite halbdurchlässige Spiegel sich in der Betriebsposition befinden, wobei die Reflexionseigenschaften der halbdurchlässigen Spiegel so gewält sind, daß etwa die gleiche Lichtmenge auf beide Kinokameras übertragen wird, durch eine Einrichtung zur Synchronisierung der Antriebe der Kinokameras, so daß deren Verschlüsse abwechselnd geöffnet werden und derart, daß die Verschlüsse niemals gleichzeitig offen sind, durch eine Fernsehkamera (7), die ebenfalls das optische Bild, das durch das optische System übertragen wird, empfängt, durch eine Betriebsarteinstelleinrichtung (8) zur Auswahl einer stereoskopischen Kinematographie-Betriebsart, einer monoskopischen Kinematographie-Betriebsart oder einer Fluoroskopie-Betriebsart, durch eine radiographische Steuereinrichtung (9), die durch die Einstelleinrichtung zur Steuerung des Antriebs der Kinokameras gesteuert wird und die Vorgesehen ist zur Erzeugung von Röntgenstrahl-Belichtungs-Schaltsignalen, die synchronisiert sind mit den Verschlußbewegungen der Kinokameras, und durch eine für die Röntgenröhre vorgesehene Schaltsteuereinrichtung (12), die durch die Schaltsignale betätigt wird und die Röntgenröhre so steuert, daß Röntgenstrahlen von den Brennpunkten abwechselnd abgestrahlt werden synchron mit den abwechselnd offenen Verschlüssen der Kinokameras, wenn die stereoskopische Kinematographiebetriebsweise gewählt ist.

2. Röntgengerät nach Anspruch 1, dadurch gekennzeichnet, daß der zweiten Kinokamera und der Fernsehkamera jeweils eine einstellbare Blende (4i, 4j) zugeordnet ist, die die Lichtmenge steuert, die in die entsprechende Kamera eintritt, und daß die radiographische Steuereinrichtung die Blenden entsprechend der Positionierung der halbdurchlässigen Spiegel steuert.

3. Röntgengerät nach Anspruch 1 oder 2, dadurch gekennzeichnet, daß die Schaltsteuereinrichtung einen unabhängigen Röntgenröhrengitter-Steuerschaltkreis aufweist zur unabhängigen Steuerung des Potentials auf jedem der beiden Gitter der Röntgenröhre in Abhängigkeit von den Schaltsignalen zur Durchführung der Ein-Aus-Steuerung der Strahlung aus den beiden Brennpunkten.

4. Röntgengerät nach Anspruch 3, dadurch gekennzeichnet, daß jede Röntgenröhrengitter-Steuerschaltkreis eine Tetrode ($124_l$, $124_r$) aufweist.

5. Röntgengerät nach einem der vorhergehenden Ansprüche, dadurch gekennzeichnet, daß der Konverter ein Bildverstärker ist.

FIG. I.

FIG. 2.

FIG. 3.

3

(a) SHUTTER SIGNALS FROM CINECAMERA 5

OPEN OPEN
CLOSE CLOSE

(b) X-RAY PULSES FOR THE RIGHT

(c) SHUTTER SIGNALS FROM CINECAMERA 6

OPEN OPEN
CLOSE CLOSE

(d) X-RAY PULSES FOR THE LEFT

(e) FILM OF CINECAMERA 5

FIG. 4.

(f) FILM OF CINECAMERA 6

0 051 430